# EUROPÄISCHE PATENTSCHRIFT

(11) **EP 0 624 583 B1**
(45) Veröffentlichungstag und Bekanntmachung des Hinweises auf die Patenterteilung: **23.07.1997**
(21) Anmeldenummer: 94106834.8
(22) Anmeldetag: 02.05.1994
(51) Int. Cl.: C07D 401/14, A61K 31/44

(54) **Substituierte Pyridylmethylpyridone als Angiotensin II Antagonisten**
Substituted pyridylmethylpyridone as angiotensine II antagonists
Pyridylméthylpyridones substituées comme antagonistes d'angiotensine II

(30) Priorität: 13.05.1993 DE 4316077
(43) Veröffentlichungstag der Anmeldung: 17.11.1994
(73) Patentinhaber: BAYER AG, 51368 Leverkusen (DE)
(72) Erfinder: Fey, Peter, Dr., D-42111 Wuppertal (DE); Hübsch, Walter, Dr., D-42113 Wuppertal (DE); Dressel, Jürgen, Dr., D-42477 Radevormwald (DE); Hanko, Rudolf, Dr., D-45134 Essen (DE); Krämer, Thomas, Dr., D-42111 Wuppertal (DE); Müller, Ulrich, Dr., D-42111 Wuppertal (DE); Müller-Gliemann, Matthias, Dr., D-42697 Solingen (DE); Beuck, Martin, Dr., D-40699 Erkrath (DE); Bischoff, Hilmar, Dr., D-42113 Wuppertal (DE); Wohlfeil, Stefan, Dr., D-40724 Hilden (DE); Denzer, Dirk, Dr., D-42115 Wuppertal (DE); Kazda, Stanislav, Prof. Dr., D-42115 Wuppertal (DE); Stasch, Johannes-Peter, Dr., D-42651 Solingen (DE); Knorr, Andreas, Dr., D-42651 Solingen (DE); Zaiss, Siegfried, Dr., D-42113 Wuppertal (DE)

(56) Entgegenhaltungen:
- EP-A- 0 500 297
- EP-A- 0 508 393
- EP-A- 0 530 702

## Beschreibung

Die Erfindung betrifft substituierte Pyridylmethylpyridone, Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Arylheteroarylalkyl substituierte Triazole und Imidazole sind aus den Publikationen EP 508 445, EP 503 393, EP 504 888 und US 5,128,327 als A II-Antagonisten bekannt.

In EP 500 297 werden Biphenyl-Verbindungen offenbart, die als charakteristischen Heterozyklus einen Pyridoring haben, der das Substitutionsmuster einer geradkettigen unverzweigten Alkyl-Gruppe in 6-Position und einer Carboxy- bzw. einer Alkoxycarbonyl-Gruppe in 4-Position des Pyridonrings aufweist.

Die Erfindung betrifft substituierte Pyridylmethylpyridone der allgemeinen Formel (I) 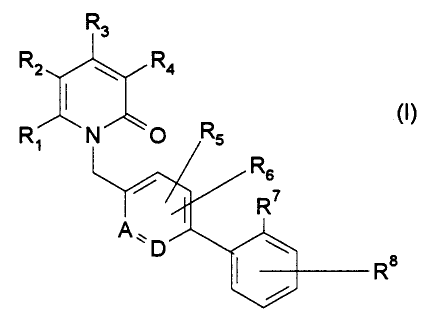 in welcher
- A oder D: für ein Stickstoffatom steht und der andere Substituiert jeweils für die CH-Gruppe steht
- R¹: für geradkettiges oder verzweigtes Alkyl mit bis zu 4 Kohlenstoffatomen steht,
- R², R³ und R⁴: gleich oder verschieden sind und
für Wasserstoff, Carboxy oder für geradkettiges oder verzweigtes Alkoxycarbonyl mit bis zu 4 Kohlenstoffatomen oder Benzyloxycarbonyl stehen,
- R⁵, R⁶ und R⁸: gleich oder verschieden sind und
für Wasserstoff, Fluor, Chlor, Brom oder Methyl stehen,
- R⁷: für eine Gruppe der Formel -CO-R¹⁴ steht,
worin
- R¹⁴: Hydroxy oder geradkettiges oder verzweigtes Alkoxy mit bis zu 4 Kohlenstoffatomen bedeutet,
oder
- R⁷: für den Tetrazolylrest der Formel 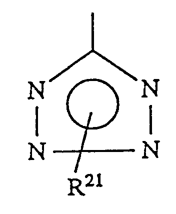 steht,
worin
- R²¹: Wasserstoff oder die Triphenylmethylgruppe bedeutet,
und deren Salze.
Die erfindungsgemäßen substituierten Pyridylmethylpyridone können auch in Form ihrer Salze vorliegen. Im allgemeinen seien hier Salze mit organischen oder anorganischen Basen oder Säuren genannt.
Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.
Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Ein 5- bis 6-gliedriger, gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidyl. Bevorzugt ist Morpholinyl.

Außerdem wurden Verfahren zur Herstellung der erfindungsgemäßen Verbindungen der allgemeinen Formel (I) gefunden, dadurch gekennzeichnet, daß man
[A] Pyridone der allgemeinen Formel (II) 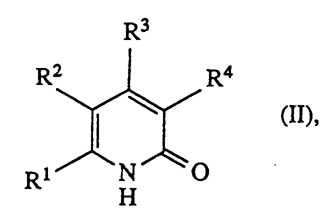 in welcher
   - R¹, R², R³ und R⁴: die oben angegebene Bedeutung haben,
   mit Verbindungen der allgemeinen Formel (III) 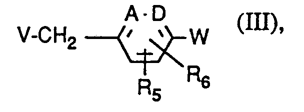 in welcher
   - A, D, R⁵ und R⁶: die oben angegebene Bedeutung haben,
   - V: für Halogen, vorzugsweise für Brom steht und
   - W: für einen Rest der Formel 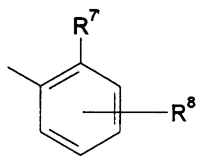 steht,
   worin
   - R⁸: die oben angegebene Bedeutung hat,
   - R^{7'}: für C₁-C₄Alkoxycarbonyl oder bevorzugt für einen Rest der Formel 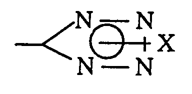 steht,
   worin
   - X: die Triphenylmethylgruppe oder Wasserstoff bedeutet,
   in inerten Lösemitteln, in Anwesenheit einer Base und gegebenenfalls unter Zusatz eines Katalysators umsetzt,
   oder
[B] Verbindungen der allgemeinen Formel (IV) 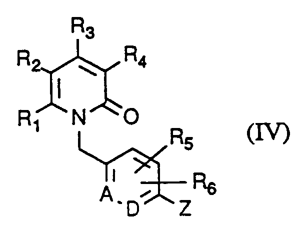 in welcher
   - R¹, R², R³, R⁴, R⁵, R⁶, A und D: die oben angegebene Bedeutung haben
   und
   - Z: für eine typische Abgangsgruppe, wie beispielsweise Brom, Jod, Methan-, Toluol-, Fluor- oder Trifluormethansulfonyloxy, vorzugsweise für Brom steht,
   mit Verbindungen der allgemeinen Formel (V) oder (Va) 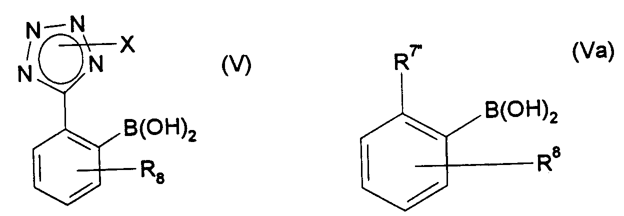 in welcher
   - R⁸ und X: die oben angegebene Bedeutung haben,
   und
   - R^{7''}: die oben angegebene Bedeutung von R⁷ hat, aber nicht für den Tetrazolylrest steht,
   in inerten Lösemitteln, in Anwesenheit einer Base und metallkatalysiert, umsetzt,
   und anschließend im Fall, X = Triphenylmethylgruppe, mit Säuren in organischen Lösemitteln und/oder Wasser nach üblichen Bedingungen abspaltet,
   und gegebenenfalls im Fall der unter den Substituenten R⁷ und/oder R^{7'} aufgeführten carbonylischen Reste, nach Verseifung der jeweiligen Ester, beispielsweise durch Amidierung oder Sulfoamidierung, nach üblichen Methoden derivatisiert,
   und im Fall der Salze bevorzugt ausgehend vom freien Tetrazol (R²¹/X = H) mit Säuren oder Basen umsetzt,
   und im Fall der freien Säure (R⁷ = CO₂H) und des freien Tetrazols (R²¹ = H), ausgehend von den Salzen, mit Säuren umsetzt,
   und gegebenenfalls auch die übrigen Substituenten nach bekannten Methoden variiert.

Das erfindungsgemäße Verfahren kann durch folgendes Formelschema beispielhaft erläutert werden:

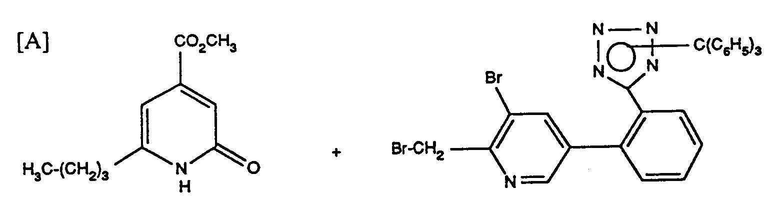

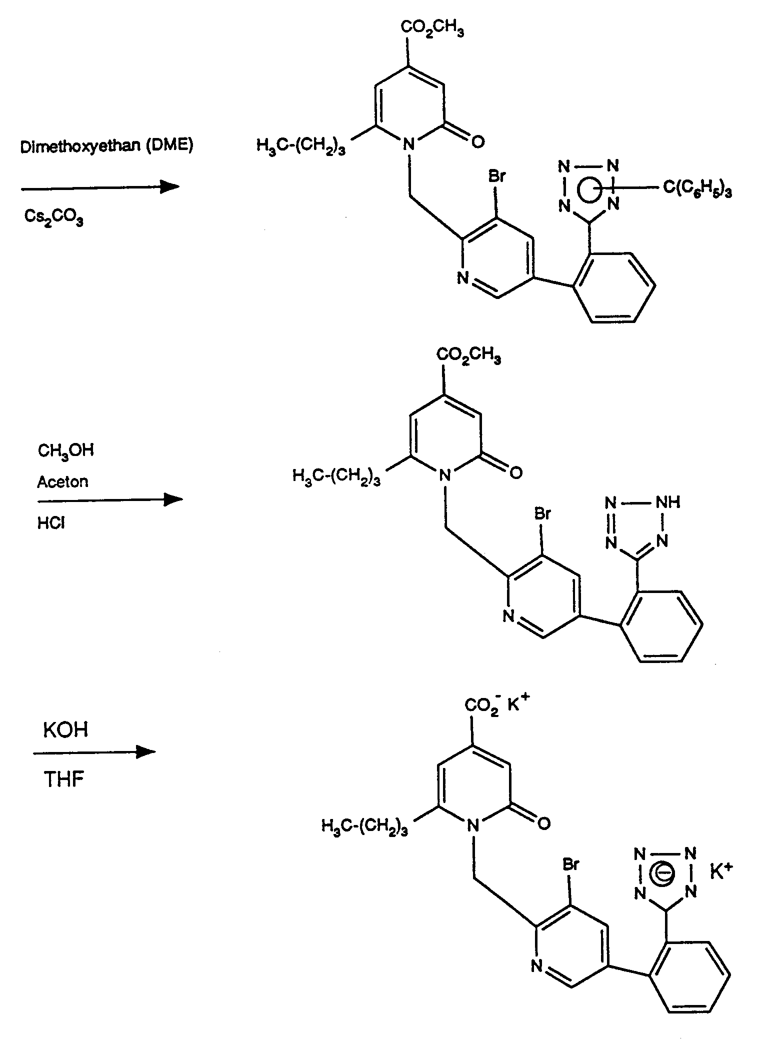

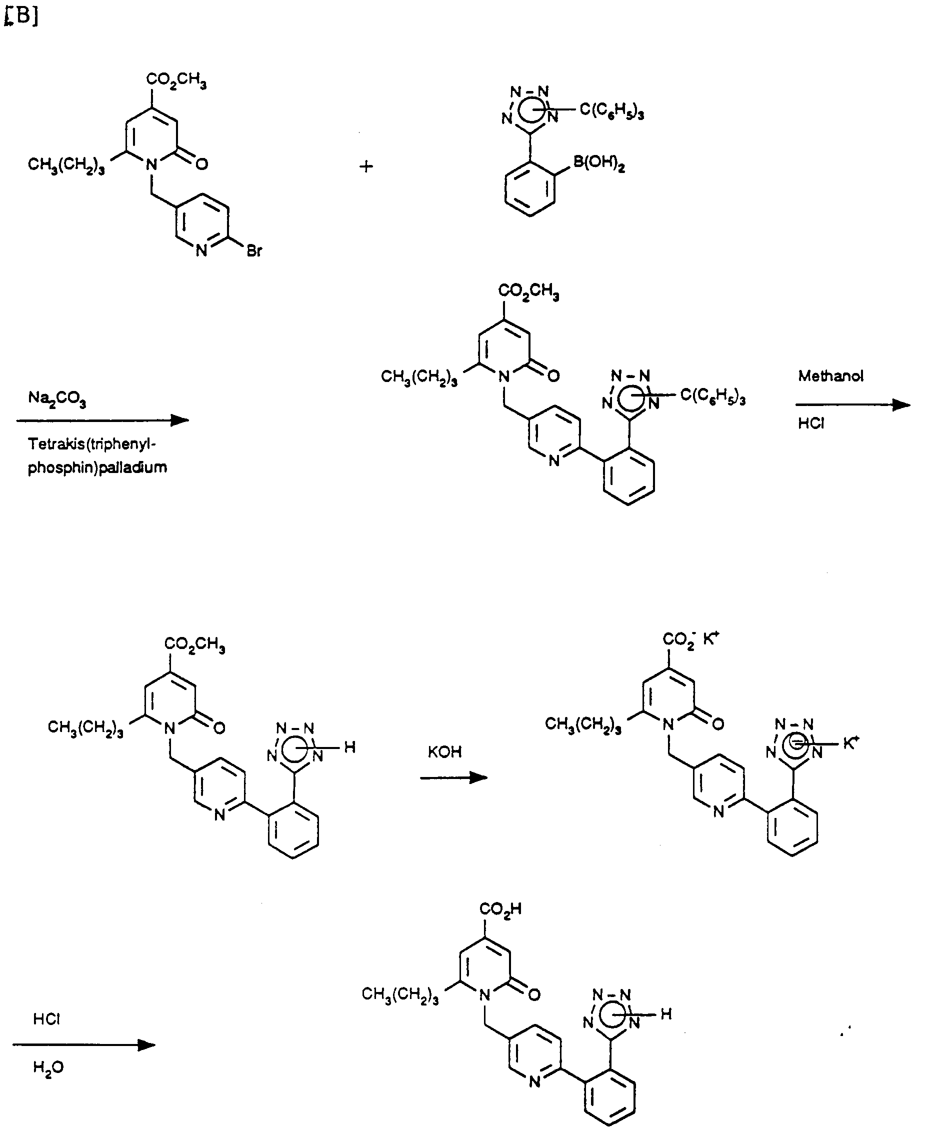

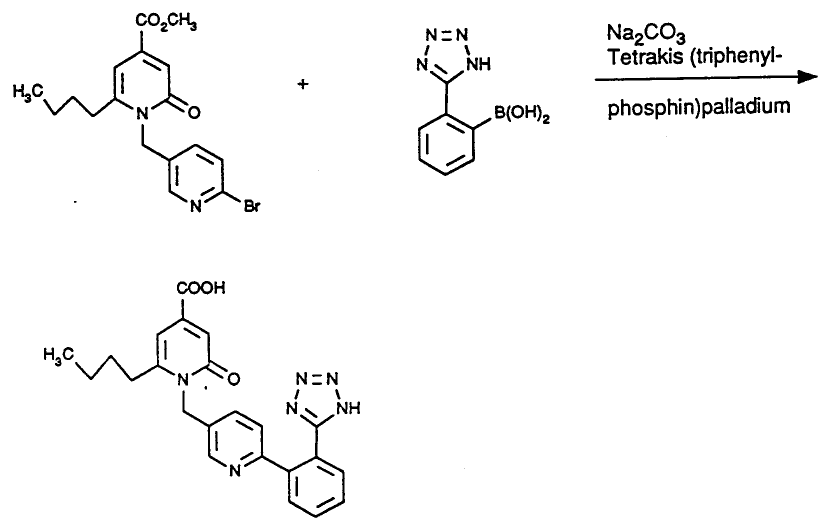

Als Lösemittel für die Verfahren [A] und [B] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt für das Verfahren [A] sind Tetrahydrofuran, Aceton, Dimethylformamid und Dimethoxyethan. Für das Verfahren [B] eignen sich außerdem noch Alkohole wie Methanol, Ethanol oder Propanol und/oder Wasser. Bevorzugt für das Verfahren [B] sind Toluol/Methanol/Wasser.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium- oder Kaliummethanolat, Natrium- oder Kaliumethanolat oder Kalium-tert.butylat, Thalliumcarbonat- oder -hydroxid, oder Lithiumdiisopropylamid (LDA), oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind für das Verfahren [A] Kaliumcarbonat, Natriumhydrid, Kalium-tert-butylat oder Caesiumcarbonat Bevorzugt für das Verfahren [B] ist Natriumcarbonat.

Im allgemeinen setzt man die Base in einer Menge von 0,05 mol bis 10 mol, bevorzugt von 1 mol bis 2 mol jeweils bezogen auf 1 mol der Verbindungen der Formel (III) oder (V) ein.

Die erfindungsgemäßen Verfahren werden im allgemeinen in einem Temperaturbereich von -100°C bis +100°C, bevorzugt von 0°C bis 80°C durchgeführt. Das erfindungsgemäße Verfahren [B] erfolgt im allgemeinen unter Schutzgasatmosphäre.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0°C bis 150°C, vorzugsweise von 20°C bis 100°C und Normaldruck.

Als Katalysatoren eignen sich für das Verfahren [A] Kalium- oder Natriumiodid, bevorzugt Natriumiodid.

Als Katalysatoren für das Verfahren [B] eignen sich im allgemeinen Metallkomplexe des Nickels, Palladiums oder Platins, bevorzugt Palladium(O)-Komplexe, wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Der Katalysator wird in einer Menge von 0,0001 mol bis 0,15 mol, bevorzugt von 0,01 mol bis 0,05 mol, bezogen auf 1 mol der Verbindung der allgemeinen Formel (V) eingesetzt.

Die Alkylierung erfolgt im allgemeinen mit Alkylierungsmitteln wie beispielsweise (C₁-C₈)-Alkylhalogeniden, Sulfonsäurestern oder substituierten oder unsubstituierten (C₁-C₈)-Dialkyl- oder (C₁-C₈)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich. von -20°C bis +80°C, vorzugsweise von -10°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonen-5 (DBN) oder 1,5-Diazabicyclo[3.4.0]undecen-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel II sind teilweise bekannt oder neu, und können in diesem Fall in Analogie zu bekannten Methoden hergestellt werden.

Die Verbindungen der allgemeinen Formel (III) sind an sich bekannt.

Die Verbindungen der allgemeinen Formel (IV) sind größtenteils neu und können beispielsweise hergestellt werden, indem man Verbindungen der allgemeinen Formel (II) mit Verbindungen der allgemeinen Formel (VI) 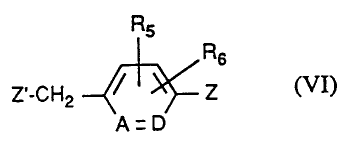 in welcher
- A, D, R⁵ und R⁶: die oben angegebene Bedeutung haben
und
- Z und Z': gleich oder verschieden sind und die oben angegebene Bedeutung von Z haben,
in einem der oben aufgeführen Lösemittel und in Anwesenheit einer der dort beschriebenen Basen, vorzugsweise in Dimethoxyethan und Cäsiumcarbonat bei Raumtemperatur umsetzt.

Die Basen werden im allgemeinen in einer Menge von 1 mol bis 5 mol, bevorzugt von 2 mol bis 3 mol, bezogen auf 1 mol der Verbindungen der allgemeinen Formel (VI) eingesetzt.

Die Verbindungen der allgemeinen Formel (VI) sind größtenteils bekannt

Die Herstellung der Verbindungen der allgemeinen Formel (IV) erfolgen im allgemeinen in einem Temperaturbereich von -100°C bis +100°C, bevorzugt von -20°C bis +30°C und Normaldruck.

Die Verbindungen der allgemeinen Formel (V) sind im Fall X = H neu und können beispielsweise hergestellt werden, indem man Phenyltetrazol zunächst unter Schutzgasatmosphäre in einem aprotischen Lösemittel und in Anwesenheit einer Base umsetzt und anschließend Borsäuretrimethylester zufügt, und in einem letzten Schritt mit Säuren hydrolysiert.

Als Lösemittel eignen sich für das Verfahren aprotische Lösemittel wie Ether, beispielsweise Tetrahydrofuran, Diethylether, Toluol, Hexan oder Benzol. Bevorzugt ist Tetrahydrofuran.

Als Basen eignen sich prim-, sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, C₁-C₄-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt von -10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (Va) sind teilweise bekannt oder nach üblichen Methoden herstellbar.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen substituierten Pyridylmethylpyridone zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrucken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Außerdem können die Verbindungen zur Bekämpfung von Glaukom, diabetischer Retinopathie Bewegungssteigerung der intraokularen Netzhautflüssigkeit eingesetzt werden.

Sie sind auch geeignet zur Bekämpfung von Erkrankungen des zentralen Nervensystems wie beispielsweise Depression, Migräne, Schizophrenie oder Angstzustanden, von Hirnleistungsstörungen, Schlaganfall, diabetischer Nephropathie, Herzrhytmusstörungen, Prophylaxe von koronaren Herzerkrankungen oder Restenoseprophylaxe nach Angioplastien und gefäßchrirugischen Maßnahmen.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2, 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### 2-Methyl-5-[(trifluoromethyl)sulfonyloxy]-pyridin

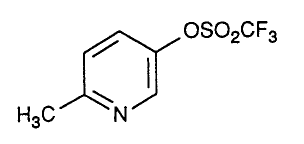

In 250 ml Dichlormethan werden 4,63 g (42,4 mmol) 3-Hydroxy-6-methylpyridin, 15,08 g (42,2 mmol) N,N-Bis-(trifluormethansulfonyl)-anilin und 6,15 ml (44,53 mmol) Triethylamin über Nacht gerührt. Das Reaktionsgemisch wird zweimal mit 1 N Natronlauge, zweimal mit wäßriger Kaliumcarbonatlösung und zweimal mit Wasser gewaschen, die organische Phase über Natriumsulfat getrocknet und am Rotationsverdampfer zu 10,47 g Öl eingeengt.
Ausbeute: 100%
¹H-NMR (CDCl₃): δ = 2,6(3H,s); 7,2(1H,m); 7,5(1H,dd); 8,47(1H,d) ppm.

### Beispiel II

### 2-Methyl-5-[2-triphenylmethyl-tetrazol-5-yl)phenyl]-pyridin

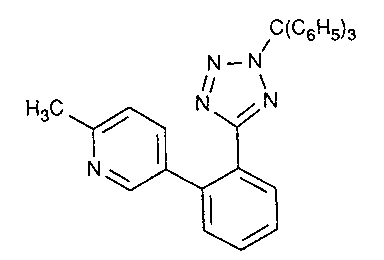

Eine Suspension aus 6,8 g (28,2 mmol) der Verbindung aus Beispiel I, 20 g (46,3 mmol) 3-(2'-Triphenylmethyl-2'H-tetrazol-5'-yl)phenylboronsäure, 3 g (28,3 mmol) Natriumcarbonat, 1,31 g (1,13 mmol) Tetrakis(triphenylphosphin)palladium, 17,5 ml Wasser, 17,5 ml Methanol und 140 ml Toluol werden unter einer Argonatmosphäre 4 h bei 90°C gerührt. Das Reaktionsgemisch wird mit Wasser verdünnt und dreimal mit Essigester gewaschen. Die organische Phase wird mit Wasser und Natriumchloridlösung gewaschen, über Natriumsulfat getrocknet, auf 150 g Kieselgel aufgezogen und an 200 g Kieselgel mit 1,6 l Essigester-Petrolethergemischen (1:10 -> 1:1)eluiert.
Ausbeute: 9,15 g Schaum (67% d.Th.)
R_{f} = 0,23 (Essigester / Petrolether 1:2, Kieselgel Si60)

### Beispiel III

### 2-Brommethyl-5-[(2-(2-triphenylmethyl-tetrazol-5-yl)phenyl]pyridin

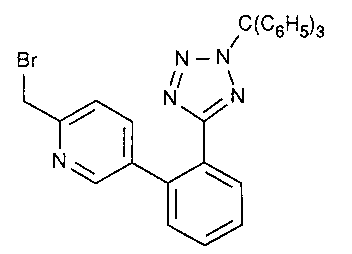

Eine Suspension aus 4,78 g (10 mmol) der Verbindung aus Beispiel II, 2 g (11,2 mmol) N-Bromsuccinimid und einer Spatelspitze 2,2'-Azo-bis(2-methylpropionsäurenitril) in 170 ml Tetrachlorkohlenstoff wird über Nacht unter Rückfluß erhitzt. Es wird vom Ungelösten abgesaugt, die Lösung eingeengt und der Rückstand an Kieselgel mit Petrolether / Essigester 5:1 zu 1,2 g der Titelverbindung chromatographiert.
Ausbeute: 21,5% der Theorie
R_{f} (Essigester / Petrolether 1:5, Kieselgel) = 0,34 Weiterhin werden 0,8 g

### Beispiel IV

### 2-Brommethyl-3-brom-5-[2-(1-triphenylmethyl-tetrazol-5-yl)phenyl]pyridin

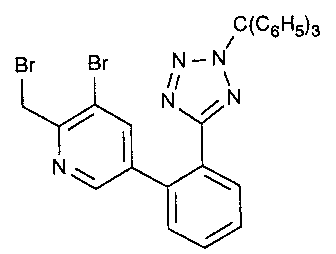

eluiert.
Ausbeute: 14% der Theorie
R_{f} (Essigester / Petrolether 1:5, Kieselgel) = 0,56

### Beispiel V

### 6-Butyl-4-methoxycarbonyl-2-oxo-1-[6-brompyridin-3-yl-methyl]-1,2-dihydropyridin

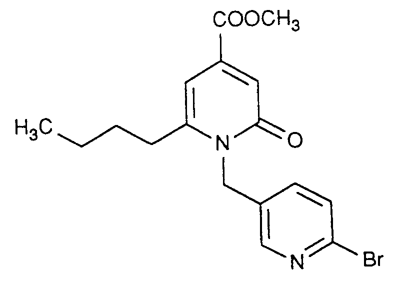

In Analogie zu Beispiel 1 werden aus 30 g (0,143 mol) 6-Butyl-4-methoxycarbonyl-2-oxo-1,2-dihydropyridin, 45,5 g (0,17 mol) 2-Brom-5-brommethylpyridin und 55,9 g (0,17 mol) Cäsiumcarbonat in 0,6 l Dimethoxyethan 17,3 g der Titelverbindung erhalten.
Ausbeute: 33,3% der Theorie
R_{f} (Essigester / Petrolether 1:2) = 0,14

### Beispiel VI

### 6-Butyl-4-carboxy-2-oxo-1-[6-brompyridin-3-ylmethyl]-1,2-dihydropyridin

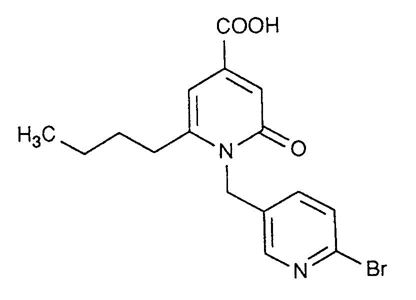

In Analogie zur Vorschrift des Beispiels 13 wird die Titelverbindung aus der Verbindung des Beispiels V erhalten:
Ausbeute: 100 %
R_{f} (Acetonitril/Wasser 5:1, Kieselgel) = 0,47

### Beispiel VII

### 4-Benzyloxycarbonyl-6-butyl-2-oxo-1-[6-brompyridin-3-yl-methyl]-1,2-dihydropyridin

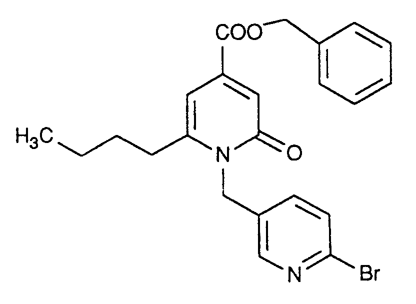

Eine Suspension aus 3,3 g (9 mmol) der Verbindung aus Beispiel VI, 0,1 g Dimethylaminopyridin, 4,7 ml (45 mmol) Benzylalkohol und 2,05 g (9,9 mmol) Dicyclohexylcarbodiimid in 50 ml Dichlormethan wird über Nacht bei 0°C gerührt. Der ausgefallene Niederschlag wird abgesaugt. daß Filtrat eingeengt und der erhaltene Rückstand an 200 g Kieselgel mit Essigester/Petrolethergemischen 1:5 - 1:1,= 3,4 g der Titelverbindung chromatographiert.
Ausbeute: 82,3 % der Theorie
R_{f} (Essigester / Petrolether 1:2, Kieselgel) = 0,25

### Beispiel VIII

### 2-(Tetrazol-5-yl)phenylboronsäure

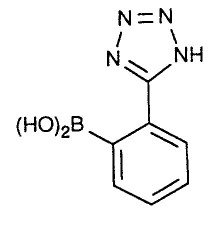

Eine Lösung von 2,9 g (20 mmol) 5-Phenyltetrazol in 50 ml THF wird bei -5°C unter Argon mit 17,6 ml (44 mmol) einer 2,5 M Lösung von n-Butyllithium in n-Hexan versetzt. Man läßt 30 min bei -5°C bis 0°C rühren und gibt bei dieser Temperatur 10 ml (88 mmol) Borsäuretrimethylester hinzu. Dann wird das Kühlbad entfernt, und die Lösung bei Raumtemperatur mit 10 ml halbkonzentrierter Salzsäure versetzt. Nach 1 h wird mit 100 ml Essigester extrahiert, die organische Phase abgetrennt und die wäßrige Phase zweimal mit je 20 ml Essigester extrahiert Die vereinigten organischen Phasen werden über Natriumsulfat getrocknet, eingeengt und der Rückstand an Kieselgel mit Toluol / Eisessig / Methanol (38 : 0,1 : 2) gereinigt.
Ausbeute: 2,65 g (70% d.Th.)
R_{f} = 0,26 (Toluol/Methanol/Eisessig = 32:8:1)
¹³C-NMR: δ = 156,7; 137,9; 133,5; 129,8; 128,9; 127,7; 126,9 ppm.

### Herstellungsbeispiele

### Beispiel 1

### 6-Butyl-4-methoxycarbonyl-2-oxo-1-{5-[2-(triphenylmethyl-tetrazol-5-yl)phenyl]-pyridin-2-ylmethyl}-1,2-dihydropyridin

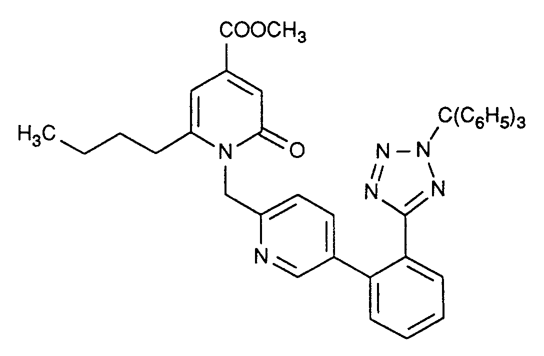

Eine Suspension von 1,1 g (1,97 mmol) der Verbindung aus Beispiel III, 374 mg (1,79 mmol) 6-Butyl-4-methoxycarbonyl-2-oxo-1,2-dihydropyridin und 642 mg (1,97 mmol) Cäsiumcarbonat werden in 15 ml Dimethoxyethan über Nacht gerührt. Das Reaktionsgemisch wird mit Wasser verdünnt und dreimal mit Essigester gewaschen. Die organische Phase wird mit Wasser und Natriumchloridlösung gewaschen, mit Natriumsulfat getrocknet und an 70 g Kieselgel mit Essigester / Petrolether (1:2) zu 45 mg Öl chromatographiert.
Ausbeute: 3,3% der Theorie
R_{f} (Kieselgel, Essigester / Petrolether 1:1) = 0,40

### Beispiel 2

### 6-Butyl-2-oxo-1-{6-[2-triphenylmethyl(tetrazol-5-yl)-phenyl]-pyridin-3-ylmethyl}-1,2-dihydropyridin-4-carbonsäuremethylester

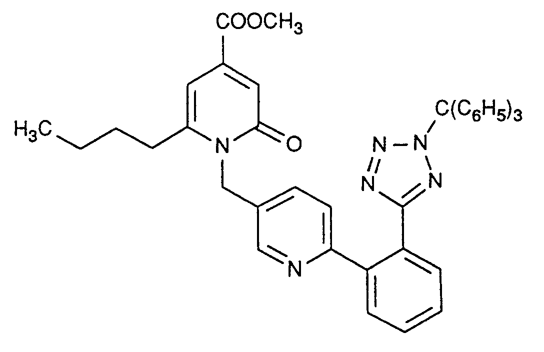

In Analogie zu Beispiel 1 werden aus 1,98 g (5,55 mmol) der Verbindung aus Beispiel V und 2,59 g (6 mmol) 3-(2'-Triphenylmethyl-2'-H-tetrazol-5'-yl)phenyl-boronsäure 2,15 g der Titelverbindung erhalten.
Ausbeute: 56% der Theorie
R_{f} (Kieselgel, Essigester) = 0,64

### Beispiel 3

### 6-Butyl-2-oxo-1-{5-[2-(1H-tetrazol-5-yl)-phenyl]pyridin-2-ylmethyl}-1,2-dihydropyridin-4-carbonsäuremethylester

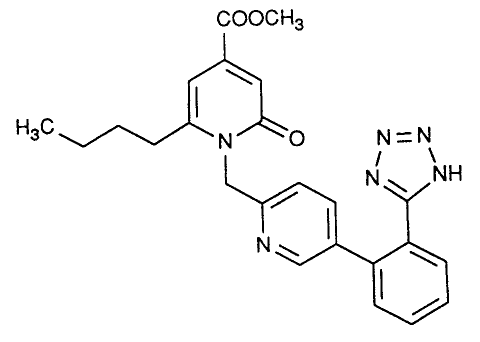

In 2 ml Methanol werden 40 mg (0,06 mmol) der Verbindung aus Beispiel 1 mit einem Tropfen konz. Salzsäure versetzt und 2 h bei Raumtemperatur gerührt. Das Reaktionsgemisch wird anschließend an 30 g Kieselgel mit Dichlormethan / Methanol-Gemischen (10:1, 5:1, 0:1) zu 18,6 mg der Titelverbindung chromatographiert.
Ausbeute: 72,4% der Theorie
MS (FAB): 444 (M⁺), 445 (M⁺+1)

### Beispiel 4

### 6-Butyl-2-oxo-1-{5-[2-(1H-tetrazol-5-yl)phenyl]pyridin-2-ylmethyl}-1,2-dihydropyridin-4-carbonsäure-dikaliumsalz

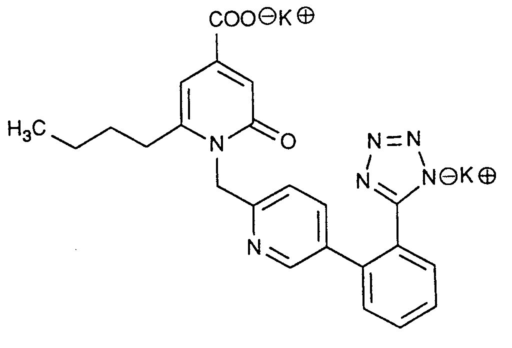

In 2 ml Tetrahydrofuran und 0,6 ml 0,1 N wässriger Kaliumhydroxidlösung werden 13,5 mg (0,03 mmol) der Verbindung aus Beispiel 3 bei 20°C 2 h gerührt. Das Lösemittel wird abdestilliert, der verbleibende Rückstand lyophilisiert und im Vakuum über Phosphorpentoxid zu 19,8 g der Titelverbindung getrocknet.
Ausbeute: 100% d.Th.
MS (FAB): 429 (M⁺), 507 (M⁺+1+2K)

Analog werden die in Tabelle 1 aufgeführten Verbindungen hergestellt:

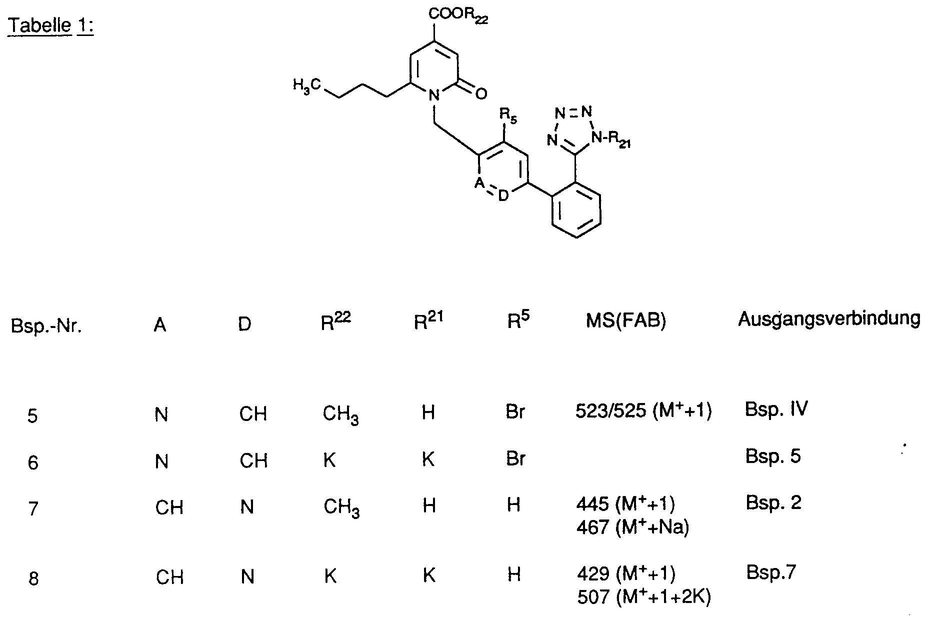

### Beispiel 9

### 6-Butyl-2-oxo-1-{6-[2-(1H-tetrazol-5-yl)phenyl]pyridin-3-ylmethyl}-1,2-dihydropyridin-4-carbonsäure-hydrochlorid.

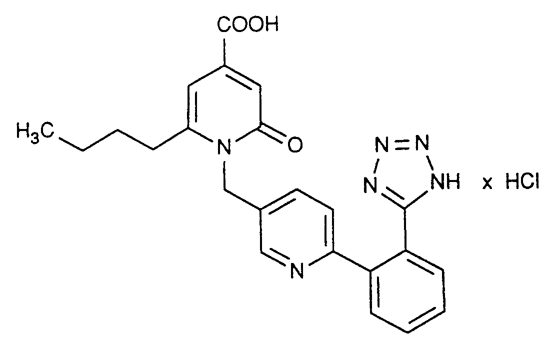

Eine Lösung von 28,5 mg (0,056 mmol) der Verbindung aus Beispiel 8 in 5 ml Wasser wird mit 1,69 ml 0,1 N Salzsäure versetzt und zu 21,8 mg der Titelverbindung lyophilisiert.
Ausbeute: 82,9% d.Th.
MS (FAB): 431

### Beispiel 10

### 6-Butyl-2-oxo-1-{6-[2-(1H-tetrazol-5-yl)-phenyl]pyridin-3-ylmethyl}-1,2-dihydropyridin-4-carbonsäuremethylester-hydrochlorid

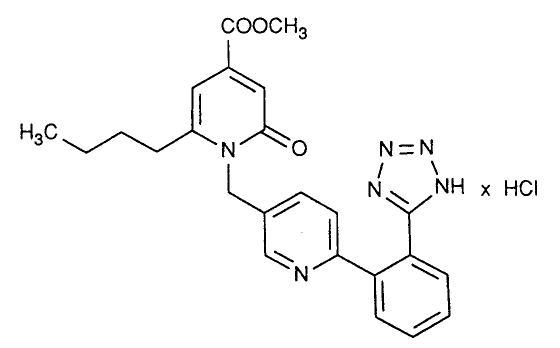

In eine Lösung von 150 mg (0,34 mmol) der Verbindung aus Beispiel 7 wird Chlorwasserstoffgas eingeleitet. Das Lösemittel wird abdestilliert und der Rückstand über Kaliumhydroxid zu 144 mg der Titelverbindung getrocknet.
Ausbeute: 88,7% d.Th.
MS (FAB): 445

### Beispiel 11

### 6-Butyl-2-oxo-1-{6-[2-(1H-tetrazol-5-yl)-phenyl]pyridin-3-ylmethyl}-1,2-dihydropyridin-4-carbonsäuremethylester-Kaliumsalz

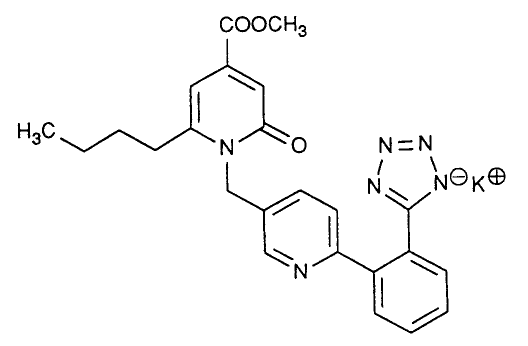

Eine Lösung von 79,3 mg (0,16 mmol) der Verbindung aus Beispiel 7 in 6 ml Methanol/3 ml Tetrahydrofuran wird mit einer Lösung von 16 mg (0,16 mmol) Kaliumhydrogencarbonat in 2 ml Wasser versetzt. Das Lösemittel wird im Vakuum bei 20°C abdestilliert und der Rückstand zu 84,4 mg der Titelverbindung lyophilisiert.
Ausbeute: 51,8% d.Th.
MS (FAB): 483

### Beispiel 12

### 6-Butyl-2-oxo-1-{5-[2-(1H-tetrazol-5-yl)-phenyl]pyridin-2-ylmethyl}-1,2-dihydropyridin-4-carbonsäuremethylester-kaliumsalz

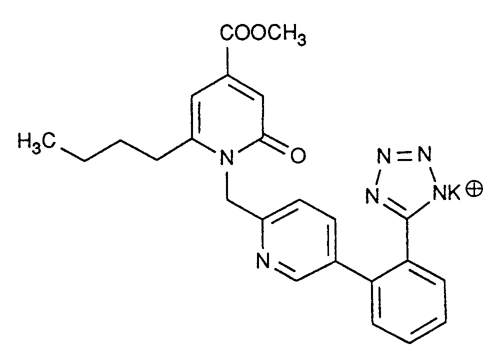

Analog der Vorschrift aus Beispiel 11 wird die Titelverbindung aus der Verbindung des Beispiels 3 erhalten.
Ausbeute: 79% der Theorie
MS (FAB): 445(M+1) 467(M+Na) 483(M+K)

### Beispiel 13

### 6-Butyl-2-oxo-1-{5-[2-(1H-tetrazol-5-yl)phenyl]pyridin-2-ylmethyl}-1,2-dihydropyridin-4-carbonsäure

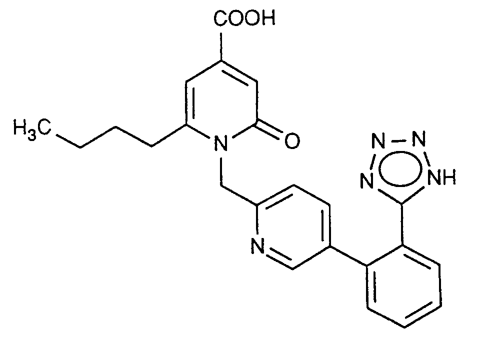

Eine Lösung von 5,9 mg (0,1 mmol) der Verbindung aus Beispiel 4 in 10 ml Wasser wird mit 1 N Salzsäure auf pH = 2 eingestellt und dreimal mit Essigester gewaschen. Die vereinigten organischen Phasen werden mit Wasser und gesättigter Natriumchloridlösung gewaschen, über Natriumsulfat getrocknet und zu 38,3 mg der Titelverbindung eingeengt.
Ausbeute: 76% der Theorie
MS (FAB): 431 (M+1)

### Beispiel 14

### 6-Butyl-2-oxo-1-{6-[2-triphenylmethyl-(tetrazol-5-yl)-phenyl]pyridin-3-ylmethyl}-1,2-dihydropyridin-4-carbonsäurebenzylester

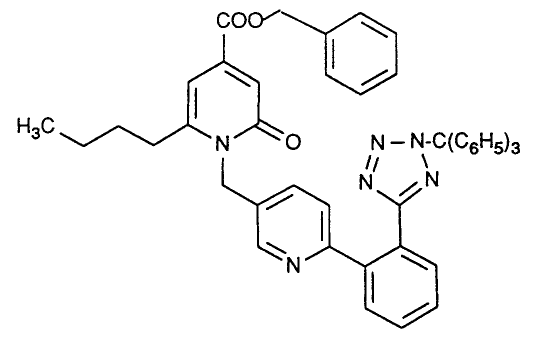

In Analogie zur Vorschrift aus Beispiel 1 werden aus 3,34 g (7,3 mmol) der Verbindung aus Beispiel VII und aus 4,1 g (8 mmol) 3-(2'-Triphenylmethyl-2'-H-tetrazol-5'-yl)phenylboronsäure 1,7 g der Titelverbindung erhalten.
Ausbeute: 31 % der Theorie
R_{f} (Essigester/Petrolether 1:1, Kieselgel) = 0,30

### Beispiel 15

### 6-Butyl-2-oxo-1-{6-[2-(1H-tetrazol-5-yl)phenyl]pyridin-3-ylmethyl}-1,2-dihydropyridin-4-carbonsäurebenzylester

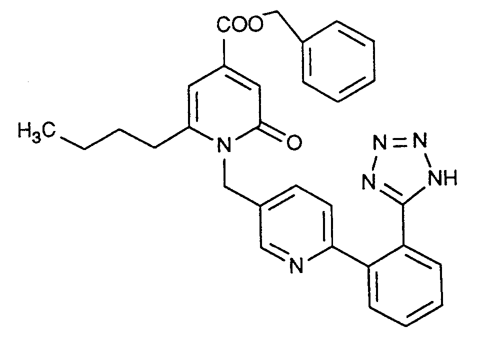

In Analogie zur Vorschrift des Beispiels 3 werden aus 1,6 g (2 mmol) der Verbindung aus Beispiel 14 1,0 g der Titelverbindung erhalten.
Ausbeute: 94% der Theorie
MS (FAB): 521 (M+H)

### Beispiel 16

### 6-Butyl-2-oxo-1-{6-[2-(1H-tetrazol-5-yl)phenyl]pyridin-3-ylmethyl}-1,2-dihydropyridin-4-carbonsäurebenzylester-kaliumsalz

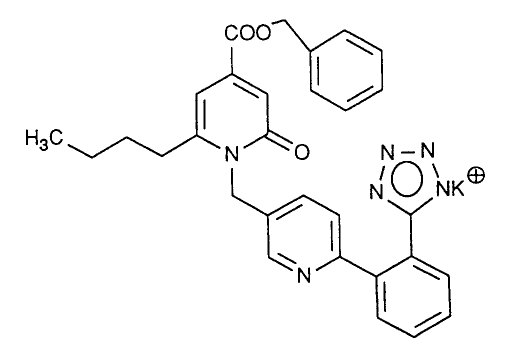

In Analogie zur Vorschrift des Beispiels 11 werden aus 948 mg (1,8 mmol) der Verbindung aus Beispiel 15 1,0g der Titelverbindung erhalten.
Ausbeute: 98,3% der Theorie
MS (FAB): 521 (M+K), 559 (M+K)

## Patentansprüche

1. Substituierte Pyridylmethylpyridone der allgemeinen Formel (I) 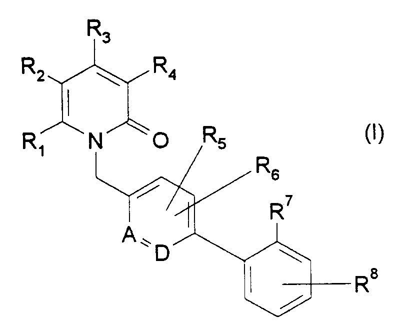 worin
A oder D für ein Stickstoffatom steht und der andere Substituiert jeweils für die CH-Gruppe steht
R¹ für geradkettiges oder verzweigtes Alkyl mit bis zu 4 Kohlenstoffatomen steht,
R², R³ und R⁴ gleich oder verschieden sind und
für Wasserstoff, Carboxy oder für geradkettiges oder verzweigtes Alkoxycarbonyl mit bis zu 4 Kohlenstoffatomen oder Benzyloxycarbonyl stehen,
R⁵, R⁶ und R⁸ gleich oder verschieden sind und
für Wasserstoff, Fluor, Chlor, Brom oder Methyl stehen,
R⁷ für eine Gruppe der Formel -CO-R¹⁴ steht,
worin
R¹⁴ Hydroxy oder geradkettiges oder verzweigtes Alkoxy mit bis zu 4 Kohlenstoffatomen bedeutet,
oder
R⁷ für den Tetrazolylrest der Formel 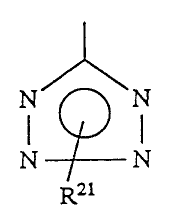 steht,
worin
R²¹ Wasserstoff oder die Triphenylmethylgruppe bedeutet,
und deren Salze.

2. Substituierte Pyridylmethylpyridone nach Anspruch 1 als Arzneimittel.

3. Verfahren zur Herstellung von substituierten Pyridylmethylpyridonen nach Anspruch 1
dadurch gekennzeichnet, daß man
[A] Pyridone der allgemeinen Formel (II) 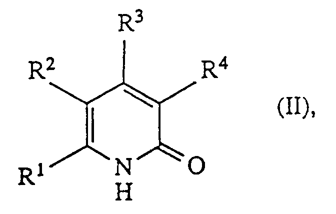 in welcher
R¹, R², R³ und R⁴ die in Anspruch 1 angegebene Bedeutung haben,
mit Verbindungen der allgemeinen Formel (III) 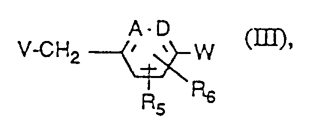 in welcher
A, D, R⁵ und R⁶ die in Anspruch 1 angegebene Bedeutung haben,
V für Halogen, vorzugsweise für Brom steht und
W für einen Rest der Formel 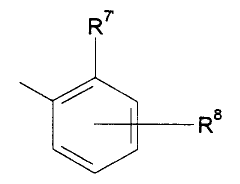 steht,
worin,
R⁸ die in Anspruch 1 angegebene Bedeutung hat,
R^{7'} für C₁-C₄Alkoxycarbonyl oder bevorzugt für einen Rest der Formel 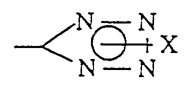 steht,
worin
X die Triphenylmethylgruppe oder Wasserstoff bedeutet,
in inerten Lösermitteln, in Anwesenheit einer Base und gegebenenfalls unter Zusatz eines Katalysators umsetzt,
oder
[B] Verbindungen der allgemeinen Formel (IV) 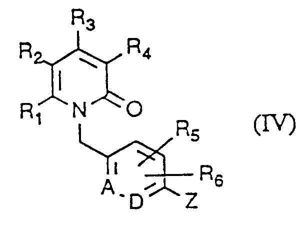 in welcher
R¹,R²_{,}R³,R⁴,R⁵,R⁶, A und D die in Anspruch 1 angegebene Bedeutung haben
und
Z für eine typische Abgangsgruppe, wie beispielsweise Brom, Jod, Methan-, Toluol-, Fluor- oder Trifluormethansulfonyloxy, vorzugsweise für Brom steht,
mit Verbindungen der allgemeinen Formel (V) oder (Va) 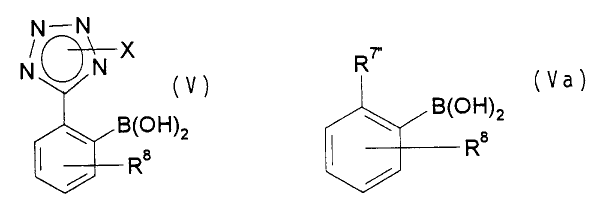 in welcher
R⁸ und X die in Anspurch 1 angegebene Bedeutung haben,
und
R^{7''} die oben angegebene Bedeutung von R⁷ hat, aber nicht für den Tetrazolylrest steht,
in inerten Lösemitteln, in Anwesenheit einer Base und metallkatalysiert, umsetzt.
und anschließend im Fall, X = Triphenylmethylgruppe, mit Säuren in organischen Lösemitteln und/oder Wasser nach üblichen Bedingungen abspaltet,
und gegebenenfalls im Fall der unter den Substituenten R⁷ und/oder R^{7'} aufgeführten carbonylischen Reste, nach Verseifung der jeweiligen Ester, beispielsweise durch Amidierung oder Sulfoamidierung, nach üblichen Methoden derivatisiert,
und im Fall der Salze bevorzugt ausgebend vom freien Tetrazol (R²¹/X = H) mit Säuren oder Basen umsetzt,
und im Fall der freien Säure (R⁷ = CO₂H) und des freien Tetrazols (R²¹ = H), ausgehend von den Salzen, mit Säuren umsetzt,

4. Arzneimittel enthaltend mindestens ein substituiertes Pyridylmethylpyridon nach Anspruch 1 sowie ein geeignetes Formulierungshilfsmittel.

5. Arzneimittel nach Anspruch 4 zur Behandlung von arterieller Hypertonie und Atherosklerose.

6. Verwendung von substituierten Pyridylmethylpyridonen nach Anspruch 1 zur Herstellung von Arzneimittel.

7. Verwendung nach Anspruch 6 zur Herstellung von Arzneimitteln zur Behandlung von arterieller Hypertonie und Atherosklerose.

## Claims

1. Substituted pyridylmethylpyridones of the general formula (I) 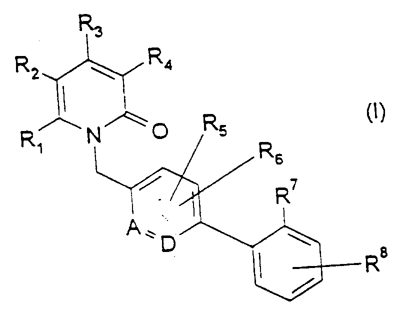 wherein
A or D represents a nitrogen atom and the other substituent in each case represents the CH group,
R¹ represents straight-chain or branched alkyl having up to 4 carbon atoms,
R², R³ and R⁴ are identical or different and
represent hydrogen, carboxyl or represent straight-chain or branched alkoxycarbonyl having up to 4 carbon atoms,
R⁵, R⁶ and R⁸ are identical or different and
represent hydrogen, fluorine, chlorine, bromine or methyl,
R⁷ represents a group of the formula -CO-R¹⁴,
wherein
R¹⁴ denotes hydroxyl or straight-chain or branched alkoxy having up to 4 carbon atoms
or
R⁷ represents the tetrazolyl radical of the formula 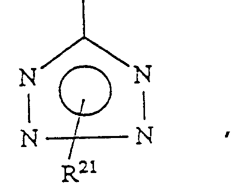 wherein
R²¹ denotes hydrogen or the triphenylmethyl group,
and salts thereof.

2. Substituted pyridylmethylpyridones according to Claim 1 as medicaments.

3. Process for the preparation of substituted pyridylmethylpyridones according to Claim 1, characterized in that
[A] pyridones of the general formula (II) 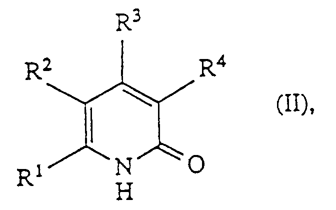 in which
R¹, R², R³ and R⁴ have the meaning mentioned in Claim 1,
are reacted with compounds of the general formula (III) 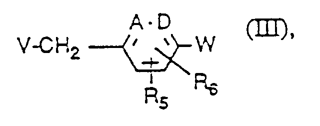 in which
A, D, R⁵ and R⁶ have the meaning mentioned in Claim 1,
V represents halogen, preferably bromine, and
W represents a radical of the formula 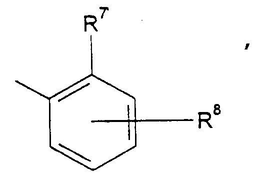 wherein
R⁸ has the meaning mentioned in Claim 1,
R^{7'} represents C₁-C₄-alkoxycarbonyl or, preferably, represents a radical of the formula 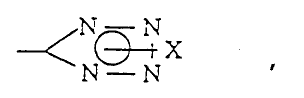 wherein
X denotes the triphenylmethyl group or hydrogen,
in inert solvents in the presence of a base and if appropriate with addition of a catalyst,
or
[B] compounds of the general formula (IV) 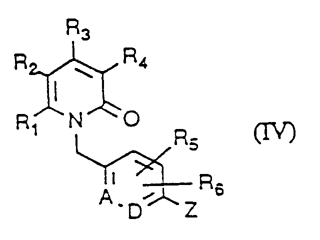 in which
R¹, R², R³, R⁴, R⁵, R⁶, A and D have the meaning mentioned in Claim 1
and
Z represents a typical leaving group, such as, for example, bromine, iodine or methane-, toluene-, fluoro- or trifluoromethanesulphonyloxy, preferably bromine,
are reacted with compounds of the general formula (V) or (Va) 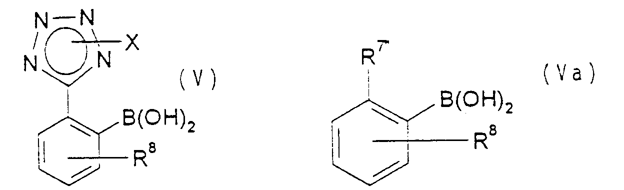 in which
R⁸ and X have the meaning mentioned in Claim 1
and
R^{7''} has the abovementioned meaning of R⁷ but does not represent the tetrazolyl radical,
in inert solvents in the presence of a base and under metal catalysis,
and, in the case where X = a triphenylmethyl group, this is subsequently eliminated with acids in organic solvents and/or water under customary conditions,
and, if appropriate, in the case of the carbonyl radicals listed under the substituents R⁷ and/or R^{7'}, derivatization is carried out by customary methods, after hydrolysis of the particular esters, for example by amidation or sulphoamidation,
and, in the case of the salts, the products are reacted with acids or bases, preferably starting from the free tetrazole (R²¹/X = H),
and, in the case of the free acid (R⁷ = CO₂H) and the free tetrazole (R²¹ = H), the products are reacted with acids, starting from the salts.

4. Medicaments comprising at least one substituted pyridylmethylpyridone according to Claim 1 and a suitable formulation auxiliary.

5. Medicaments according to Claim 4 for the treatment of arterial hypertension and atherosclerosis.

6. Use of substituted pyridylmethylpyridones according to Claim 1 for the preparation of medicaments.

7. Use according to Claim 6 for the preparation of medicaments for the treatment of arterial hyper-tension and atherosclerosis.

## Revendications

1. Pyridylméthylpyridones substituées de formule générale (I) 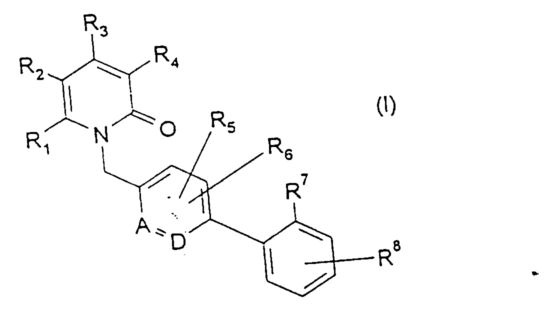 dans laquelle
A ou D représente un atome d'azote et l'autre substituant représente le groupe CH
R¹ est un groupe alkyle linéaire ou ramifié ayant jusqu'à 4 atomes de carbon,
R², R³ et R⁴ sont identiques ou différents et représentent de l'hydrogène, un groupe carboxy ou un groupe alkoxycarbonyle linéaire ou ramifié ayant jusqu'à 4 atomes de carbone ou un groupe benzyloxycarbonyle,
R⁵, R⁶ et R⁸ sont identiques ou différents et représentent de l'hydrogène, du fluor, du chlore, du brome ou un groupe méthyle,
R⁷ est un groupe de formule -CO-R¹⁴,
où
R¹⁴ est un groupe hydroxy ou un groupe alkoxy linéaire ou ramifié ayant jusqu'à 4 atomes de carbone,
ou bien
R⁷ représente le reste tétrazolyle de formule 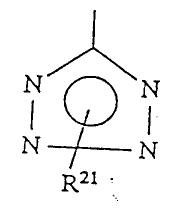 dans laquelle
R²¹ est de l'hydrogène ou le groupe triphénylméthyle,
et leurs sels.

2. Pyridylméthylpyridones substituées suivant la revendication 1, en tant que médicaments.

3. Procédé de production de pyridylméthylpyridones substituées suivant la revendication 1,
caractérisé en ce que :
[A] on fait réagir des pyridones de formule générale (II) 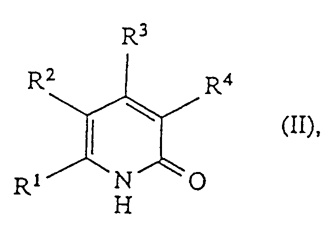 dans laquelle
R¹, R², R³ et R⁴ ont la définition indiquée dans la revendication 1,
avec des composés de formule générale (III) 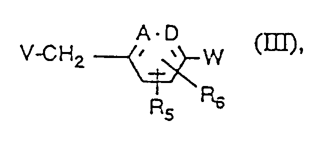 dans laquelle
A, D, R⁵ et R⁶ ont la définition indiquée dans la revendication 1,
V représente un halogène, de préférence le brome et
W est un reste de formule 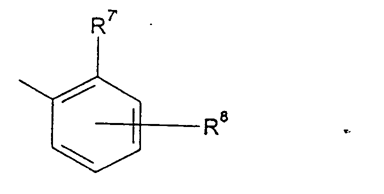 dans laquelle
R⁸ a la définition indiquée dans la revendication 1,
R^{7'} est un groupe (alkoxy en C₁ à C₄)carbonyle ou représente de préférence un reste de formule 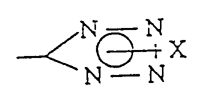 dans laquelle
X est le groupe triphénylméthyle ou de l'hydrogène,
dans des solvants inertes, en présence d'une base et, le cas échéant, avec l'addition d'un catalyseur,
ou bien
[B] on fait réagir des composés de formule générale (IV) 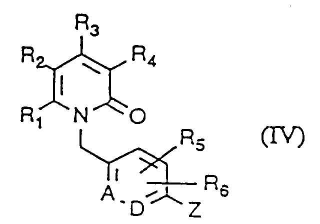 dans laquelle
R¹, R², R³, R⁴, R⁵, R⁶, A et D ont la définition indiquée dans la revendication 1,
et
Z est un groupe partant classique tel que, par exemple, le brome, l'iode, un groupe méthane-, toluène-, fluoro- ou trifluorométhanesulfonyloxy, de préférence le brome,
avec des composés de formule générale (V) ou (Va) 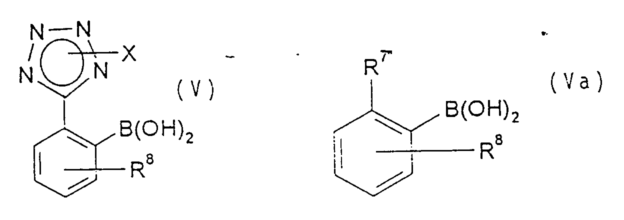 dans laquelle
R⁸ et X ont la définition indiquée dans la revendication 1,
et
R^{7''} a la définition indiquée ci-dessus pour R⁷, hormis le reste tétrazolyle,
dans des solvants inertes, en présence d'une base et d'un catalyseur métallique,
puis au cas où X représente le groupe triphénylméthyle, on l'élimine avec des acides dans des solvants organiques et/ou dans l'eau dans des conditions classiques,
et, le cas échéant, dans le cas des restes carbonyliques indiqués pour les substituants R⁷ et/ou R^{7'}, on effectue une dérivatisation par des procédés classiques après saponification des esters correspondants, par exemple par amidation ou sulfamidation,
et dans le cas des sels, on conduit la réaction avec des acides ou des bases préférentiellement à partir du tétrazole libre (R²¹/X = H)
et dans le cas de l'acide libre (R⁷ = CO₂H) et du tétrazole libre (R²¹ = H), on conduit la réaction avec des acides à partir des sels.

4. Médicament contenant au moins une pyridylméthylpyridone substituée suivant la revendication 1 ainsi qu'une substance auxiliaire de formulation appropriée.

5. Médicament suivant la revendication 4, pour le traitement de l'hypertonie artérielle et de l'athérosclérose.

6. Utilisation de pyridylméthylpyridones substituées suivant la revendication 1 pour la préparation de médicaments.

7. Utilisation suivant la revendication 6 pour la préparation de médicaments destinés au traitement de l'hypertonie artérielle et de l'athérosclérose.
